# EUROPÄISCHE PATENTSCHRIFT

(11) **EP 2 414 560 B1**
(45) Veröffentlichungstag und Bekanntmachung des Hinweises auf die Patenterteilung: **23.10.2013**
(21) Anmeldenummer: 10709554.9
(22) Anmeldetag: 22.03.2010
(51) Int. Cl.: C23C 18/12, A61M 11/00, B05B 11/00, A61M 15/00, A61M 11/06

(54) **VERFAHREN ZUR BESCHICHTUNG EINER OBERFLÄCHE EINES BAUTEILS**
METHOD FOR COATING A SURFACE OF A COMPONENT
PROCÉDÉ DE REVÊTEMENT D'UNE SURFACE D'UN COMPOSANT

(30) Priorität: 31.03.2009 EP 09156940
(43) Veröffentlichungstag der Anmeldung: 08.02.2012
(60) Teilanmeldung: 13177354.1
(73) Patentinhaber: Boehringer Ingelheim International GmbH, 55216 Ingelheim am Rhein (DE)
(72) Erfinder: SCHUY, Steffen, 55216 Ingelheim Am Rhein (DE); MEISENHEIMER, Martin, 55216 Ingelheim Am Rhein (DE); WITTE, Florian, 55216 Ingelheim Am Rhein (DE)
(74) Vertreter: Simon, Elke Anna Maria
(86) Internationale Anmeldenummer: PCT/EP2010/053668
(87) Internationale Veröffentlichungsnummer: WO 2010/112358

(56) Entgegenhaltungen:
- WO-A1-94/07607
- DE-A1- 10 300 983
- "Quantitative Online Detection of Low-Concentrated Drugs via a SERS Microfluidic System" CHEMPHYSCHEM, Bd. 8, Nr. 18, 5. Dezember 2007 (2007-12-05), Seiten 2665-2670, XP002605077
- HENKEL T ET AL: "Chip modules for generation and manipulation of fluid segments for micro serial flow processes" CHEMICAL ENGINEERING JOURNAL, ELSEVIER SEQUOIA, LAUSANNE, CH LNKD- DOI:10.1016/J.CEJ.2004.01.021, Bd. 101, 1. August 2004 (2004-08-01), Seiten 439-445, XP002594289 ISSN: 1385-8947 [gefunden am 2004-05-18] in der Anmeldung erwähnt
- HAN Y ET AL: "Surface activation of thin silicon oxides by wet cleaning and silanization" THIN SOLID FILMS, ELSEVIER-SEQUOIA S.A. LAUSANNE, CH LNKD- DOI:10.1016/J.TSF.2005.11.048, Bd. 510, Nr. 1-2, 3. Juli 2006 (2006-07-03), Seiten 175-180, XP025006930 ISSN: 0040-6090 [gefunden am 2006-07-03]
- M. WANG, K. M. LIECHTI, Q. WANG, J. M. WHITE: "Self-Assembled Silane Monolayers: Fabrication with Nanoscale Uniformity" LANGMUIR, Bd. 21, Nr. 5, 1. März 2005 (2005-03-01), Seiten 1848-1857, XP002605078

## Beschreibung

Die Erfindung bezieht sich auf ein Verfahren zur Beschichtung einer, insbesondere mikrostrukturierten, Oberfläche eines aus unterschiedlichen Werkstoffen, insbesondere Glas und Silizium, bestehenden Bauteils, bei dem zunächst die Oberfläche aktiviert und anschließend beschichtet wird, ein beschichtetes Bauteil und einen Zerstäuber mit einem beschichteten Bauteil.

In der WO 91/14468 A1 sowie der WO 97/12687 A1 ist jeweils ein Zerstäuber dargestellt, der unter dem Handelsnamen "Respimat" von der Boehringer Ingelheim Pharma GmbH & Co. KG in Form eines Inhalators angeboten wird. Der Zerstäuber dient zur treibgasfreien Verabreichung einer dosierten Menge eines flüssigen Arzneimittels zur inhalativen Anwendung. In dem Inhalator werden flüssige Arzneistoffformulierungen in einem Reservoir gelagert und über ein Steigrohr in eine Dosierkammer befördert, um schließlich durch eine Düse auszutreten.

Die Düse weist eine Flüssigkeitseinlassseite und eine Flüssigkeitsauslassseite auf. Auf der Flüssigkeitseinlassseite befindet sich eine Öffnung, durch die das von der Dosierkammer kommende flüssige Arzneimittel in die Düse eintreten kann. Auf der gegenüberliegenden Seite, der freien Stirnseite der Düse, tritt die Flüssigkeit dann durch zwei Düsenöffnungen aus, die so ausgerichtet sind, dass die aus den Öffnungen austretenden Flüssigkeitsstrahlen aufeinanderprallen und dadurch zerstäubt werden. Die Düsenöffnungen sind in mindestens zwei aufeinanderliegenden Platten ausgebildet, von denen wenigstens eine eine Mikrostruktur aufweist, so dass die beiden fest miteinander verbundenen Platten auf einer Seite einen Flüssigkeitseinlass definieren, dem sich ein Kanal- und/oder Filtersystem anschließt, das in die Düsenöffnungen mündet. Die beiden Platten mit der Mikrostruktur und den Düsenöffnungen werden im Zusammenhang mit der Respimat-Technologie als Uniblock bezeichnet.

Ein derartiger Zerstäuber bringt zumeist Formulierungen auf Basis von Wasser oder Wasser-Ethanol-Gemischen aus und kann innerhalb kurzer Zeit eine kleine Menge der flüssigen Arzneistoffformulierung in der therapeutisch notwendigen Dosierung in ein therapeutisch-inhalativ geeignetes Aerosol vernebeln. Mit dem Zerstäuber können Mengen von weniger als 100 Mikroliter mit beispielsweise einem Hub zu einem Aerosol mit einer durchschnittlichen Teilchengröße von weniger als 20 Mikrometern so vernebelt werden, dass der inhalierbare Anteil des Aerosols bereits der therapeutisch wirksamen Menge entspricht.

In dem Zerstäuber mit Respimat-Technologie wird die Arzneimittellösung mittels eines hohen Drucks von bis zu 300 bar in eine lungengängige Aerosolwolke niedriger Geschwindigkeit überführt, die dann vom Patienten eingeatmet werden kann. In seltenen Fällen können sich Rückstände aus der Formulierungslösung während der Benutzung des Zerstäubers als Verunreinigung an den Düsenauslässen anhaften, akkumulieren und zu einer Verlegung der Düsenauslässe führen, womit eine Ablenkung der Flüssigkeitsstrahlen und eine Veränderung des Feinpartikelanteils einhergehen kann. Auch wenn dieser Effekt bei einem Zerstäuber der Respimat-Technologie sehr selten ist, ist es aus Gründen der Qualitätssicherung ein Anliegen, Ablagerungen zu vermeiden.

Zur Vermeidung einer Verunreinigung einer Außenoberfläche des Düsensystems oder eines Mundstückes durch sich niedergeschlagene Flüssigkeit mit Feinpartikelanteilen schlägt die DE 103 00 983 A1 vor, dass die entsprechenden Flächen zumindest teilweise mikro- oder nanostrukturiert sind. Diese Strukturierung wird dabei dadurch erzeugt, dass die Strukturen bereits bei der Herstellung aus hydrophoben Materialien oder nachträglich durch subtraktive oder additive Behandlung der Oberflächen geschaffen werden. Als Beispiele für solche Strukturierungen werden unter anderem eine strukturierte Hydrophobierung mit nachträglicher Silanisierung, das Aufbringen von Strukturen aus hydrophoben Polymeren, die Beschichtung mit Silikonfarbe und das Aufbringen einer Polyesterfolie mit strukturierter Acrylschicht genannt.

Bzgl. der Vermeidung von Anlagerung von Silber-Colloid-Nanopartikel-Aggregaten an gläsernen Oberflächen offenbaren Ackermann et al. im Artikel "Quantitative Online Detection of Low-Concentrated Drugs via a SERS Microfluidic System"(erschienen in CHEMPHYSCHEM 2007, Band 8, Seiten 2665-2670) einen gläsernen Chip mit Mikrokanälen, die durch anodisches Bonden zweier spiegelbildlich mikrostrukturierten Substrate gebildet werden. Eine für den Bondprozess aufgebrachte 100 Nanometer Siliziumschicht wird durch Behandlung mit NaOH-Lösungen und Wasserspülung aus den Mikrokanälen entfernt. Zur Schaffung einer lipophilen Mikrokanal-Oberfläche wird der Mikrochip mittels einer Behandlung mit einer Mischung aus Schwefelsäure und wässriger Wasserstoff-Peroxid-Lösung aktiviert, mit Wasser gewaschen, getrocknet und anschließend mit einer wasserfreien Oktadecyl-Trichlorsilan in Tetradecan gespült.

Es ist Aufgabe der Erfindung, ein Verfahren und ein Bauteil sowie einen Zerstäuber mit einem Bauteil der eingangs genannten Art zu schaffen, das bzw. der selbstreinigend mit einer dünnen Funktionalisierungsschicht ausgebildet ist.

Erfindungsgemäß wird die Aufgabe bei dem Verfahren dadurch gelöst, dass zur Aktivierung der Oberfläche eine oxidierende Lösung, eine basische Lösung oder eine sauer-oxidierende Lösung verwendet wird.

Überraschenderweise wurde festgestellt, dass die zuvor erwähnten Lösungen zur Aktivierung von Oberflächen von zwei verschiedenen Materialien, insbesondere Silizium und Glas, verwendet werden können, die zu einem monolithischen Bauteil gefügt sind. In der Literatur wird stets eine spezielle Lösung einem bestimmten Werkstoff zugeordnet, um die für eine Grenzflächenfunktionalisierung benötigten reaktiven Gruppen zu erzeugen, da die Aktivierung auf das jeweilige Substrat angepasst und spezifisch für unterschiedliche Materialien entwickelt werden muss. Beispielsweise erfolgt die Aktivierung der Oberfläche des Bauteils mit einer so genannten Piranha-Lösung, nämlich der stark oxidierend wirkenden Peroxomonoschwefelsäure (konzentrierte Schwefelsäure: Wasserstoffperoxid-Lösung (30%) im Verhältnis 7:3), bei 70°C für 20 min. Alternativ kann die RCA-Reinigung angewandt werden, die üblicherweise bei der Wafer-Reinigung in der Mikroelektronik zum Einsatz kommt. Hierbei wird die folgende Lösung verwendet: Wasser:Ammoniak-Lösung (25%):Wasserstoffperoxid-Lösung (30%), Im Verhältnis 5:1:1. Das Bauteil wird der Lösung bei 75°C für 20 min. ausgesetzt.

Bevorzugt wird zur Aktivierung der Oberfläche als basische Lösung ein Küvettenreiniger verwendet. Vorzugsweise kommt der unter dem Handelsnamen "Hellmanex-Lösung" bekannte Küvettten- oder Glasreiniger der Firma Hellma GmbH & Co. KG, Müllheim, Deutschland, als wässrige 2%ige Lösung bei 70°C für 2x20 min im Ultraschallbecken zum Einsatz, wobei der Hersteller von dem Einsatz von Ultraschall im Zusammenhang mit seiner "Hellmanex-Lösung" abrät. Die "Hellmanex-Lösung" stellt eine Aktivierung sicher, die auf die unterschiedlichen Materialien, insbesondere Silizium und Glas, zur Erzeugung reaktiver Grenzflächen wirkt.

Zweckmäßigerweise wird die Oberfläche vor der Aktivierung gereinigt, insbesondere mittels Isopropanol und Wasser. Das Bauteil wird 5 min in Isopropanol und 5 min in Wasser getaucht. In Ausgestaltung wird die Oberfläche nach der Reinigung in einer Flusssäurelösung behandelt. Das Baden erfolgt für ca. 20 min in einer wässrigen 3% Flusssäurelösung (HF).

Nach einer Weiterbildung werden zur Beschichtung der Oberfläche funktionelle Silane in unpolaren, aprotischen oder polaren, protischen Lösungsmitteln verwendet. Die Beschichtung ist sehr dünn und erfolgt in einer Monolage. Das Bauteil mit seinen aktivierten Oberflächen wird bei einer toluolischen Reaktionsführung ca. 2 h in eine 0,1 bis 1% Lösung von funktionellen Tricholosilane oder Dimethylmonochlorsilane in trockenen unpolaren, aprotischen Lösungsmitteln, wie Toluol, Benzol, Tetrachlorkohlenstoff, n-Alkane (C5-C10) oder dergleichen, eingetaucht. Anschließend wird das Bauteil in einem Stickstoffstrom getrocknet und ca. 1h bei etwa 120°C in einem Ofen getempert. Überschüssige und nicht kovalent gebundene Rückstände werden nach dem Tempern mittels Toluol und Isopropanol von dem Bauteil gewaschen. Bei einer alkoholischen, insbesondere isopropanolischen, Reaktionsführung muss zunächst eine Reaktionslösung aktiviert werden, um eine Grenzflächenfunktionalisierung des Bauteils mit seinen aktivierten Oberflächen aus Silizium und Glas vornehmen zu können. Eine 0,1 bis 1% Lösung von funktionellen Triethoxysilanen in 2-Propanol/Wasser/Salzsäure (HCL) in einem Verhältnis 90 bis 98:2 bis 10:0,2 bis 0,5 wird ca. 5h bei Raumtemperatur gerührt. Anschließend wird das Bauteil mit seinen aktivierten Oberflächen für ca. 2h in die Lösung getaucht. Nach der Entnahme des Bauteils aus der Lösung wird die überschüssige Lösung schonend von dem Bauteil entfernt, was beispielsweise durch Abtropfen über einem saugfähigen Flies erfolgen kann. Danach wird das Bauteil bei etwa 120°C für ca. 2h getempert. Überschüssige und nicht kovalent gebundene Rückstände werden nach dem Tempern mittels Isopropanol und Wasser von dem Bauteil gewaschen. Zur Beschichtung der Oberflächen werden die folgenden Stoffklassen eingesetzt: Perflouroalkylsilane, Alkylsilane, Aminoalkylsilane, Carbonsäurensilane, Trimethylammoniumsilane mit unterschiedlicher Kettenlänge, insbesondere C4-C18, wobei als funktionelle Silane reaktive Trihalogensilane (R1-Si-R₃, mit R=Cl, Br), Monohalogendimethylsilane (R1-Si(CH₃)₂R, mit R=Cl, Br) oder Trialkoxysilane (R1-Si-R₃, mit R= Methoxy- oder Ethoxy-Gruppen) verwendet werden.

Um eine gute Benetzung der zu beschichtenden Oberflächen des Bauteils sicherzustellen, wird vorteilhafterweise zur Beschichtung der Oberflächen das Bauteil in der Reaktionslösung mit Ultraschall behandelt. Durch die Ultraschallbehandlung wird insbesondere der Austausch der Reaktionslösung in den Kapillaren des Bauteils bewirkt.

Die Aufgabe wird bei dem Bauteil, das mindestens zwei fest miteinander verbundene Platten umfasst, von denen wenigstens eine Platte eine Mikrostruktur zur Ausbildung eines Kanal- und/oder Filtersystems und mindestens einer sich daran anschließenden Düsenöffnung aufweist, dadurch gelöst, dass zumindest die Oberfläche der Mikrostruktur eine Beschichtung aufweist, die in dem zuvor erläuterten Verfahren aufgebracht ist.

Durch diese Maßnahme ist eine Wechselwirkung von Bestandteilen und Partikeln der durch die Düsenöffnung ausgetragenen Formulierung mit den Grenzflächen des Bauteils unterbunden. Die freie Energie der Oberfläche und somit die Benetzbarkeit ist in diesem Bereich minimiert, womit eine Verminderung der Immobilisierung von Materialrückständen am Düsenauslass, also unmittelbar im Bereich der Düsenöffnung, einhergeht. Bei der Verwendung des Bauteils werden die Materialrückstände aus dem Bauteil ausgetragen und das beschichtete Bauteil ist durch den Einsatz der monomolekularen, kovalent gebundenen Antihaftschicht selbstreinigend. Die Funktionalisierung kann in Abhängigkeit von der Wechselwirkung unterschiedlicher Formulierungsbestandteile mit den Grenzflächen des Bauteils variabel geändert werden und beispielsweise hydrophile oder hydrophobe, positiv oder negativ geladene, oleophile oder oleophobe Eigenschaften aufweisen.

Zweckmäßigerweise besteht die eine Platte aus Silizium und die andere Platte aus Glas. Die Platte aus Silizium ist mit der Mikrostruktur versehen, weist also das Kanal- bzw. Filtersystem und die Düsenöffnungen auf und ist mit der Platte aus Glas verklebt.

Das Bauteil besteht aus den beiden fest miteinander verbundenen Platten aus Glas und Silizium, wobei die aus Silizium bestehende Platte einen oder mehrere mikrostrukturierte Kanäle aufweist, die die Einlassseite mit der Auslassseite verbinden. Auf der Auslassseite kann mindestens eine runde oder nicht-runde Düsenöffnung von 2 bis 10 µm Tiefe und 5 bis 15 µm Breite sein, wobei die Tiefe bevorzugt bei 4, 5 bis 6,5 µm und die Länge bei 7 bis 9 µm beträgt. Im Fall von mehreren Düsenöffnungen, bevorzugt sind zwei, können die Strahlrichtungen der Düsen im Bauteil, das im Wesentlichen einen Düsenkörper bildet, parallel zueinander verlaufen oder sie sind in Richtung Düsenöffnung gegeneinander geneigt. Bei einem Bauteil mit mindestens zwei Düsenöffnungen auf der Auslassseite können die Strahlrichtungen mit einem Winkel von 20° bis 160° gegeneinander geneigt sein, bevorzugt wird ein Winkel von 60° bis 150°, insbesondere bevorzugt 70° bis 100°. Die Düsenöffnungen sind bevorzugt in einer Entfernung von 10 bis 200 µm angeordnet, stärker bevorzugt in einer Entfernung von 10 bis 100 µm, besonders bevorzugt 30 bis 70 µm. Am stärksten bevorzugt sind 50 µm. Die Strahlrichtungen treffen sich dementsprechend in der Umgebung der Düsenöffnungen. Der Einfachheit halber wird im Folgenden eine Ausführungsform beschrieben, bei der lediglich die aus Silizium bestehende Platte des Bauteils reliefartige Mikrostrukturen aufweist, nicht jedoch die aus Glas bestehende Platte. In anderen Ausführungsformen ist die Situation gerade umgekehrt oder beide Platten weisen diese Mikrostrukturen auf. Auf der Silizium-Platte kann auf der ebenen Oberfläche ein Satz von Kanälen ausgebildet sein, um im Zusammenwirken mit der im Wesentlichen ebenen Oberfläche der Glas-Platte eine Vielzahl von Filterdurchgangswegen zu schaffen (Filterkanäle). Daneben kann die Silizium-Platte eine Plenumkammer aufweisen, deren Decke wiederum durch die Glas-Platte gebildet ist. Die Plenumkammer kann den Filterkanälen vor- oder nachgeschaltet sein. Es können auch zwei derartige Plenumkammern ausgebildet sein. Ein anderer Satz von Kanälen auf der im Wesentlichen ebenen Oberfläche der Silizium-Platte, der den Filterkanälen nachgeschaltet ist, bildet zusammen mit der Glas-Platte einen Satz von Kanälen, die eine Vielzahl von Düsenauslassdurchgangswegen schaffen. Bevorzugt liegt der Gesamtquerschnittsflächenbereich der Düsenauslässe bei 25 bis 500 µm². Der gesamte Querschnittsflächenbereich beträgt bevorzugt 30 bis 200 µm². In einer anderen Ausführungsform weist auch diese Düsenkonstruktion nur eine einzige Düsenöffnung auf. In anderen Ausführungsformen dieser Art fehlen die Filterkanäle und/oder die Plenumkammer. Bevorzugt werden die Filterkanäle durch Vorsprünge gebildet, die zickzackförmig angeordnet sind. So bilden beispielsweise mindestens zwei Reihen der Vorsprünge eine solche Zick-Zack-Konfiguration. Auch können mehrere Reihen von Vorsprüngen ausgebildet sein, wobei die Vorsprünge jeweils seitlich zueinander versetzt sind, um dadurch zu diesen Reihen windschiefe weitere Reihen aufzubauen, wobei dann diese zuletzt beschriebenen Reihen die Zick-Zack-Konfiguration bilden. In solchen Ausführungsformen kann der Einlass und der Auslass jeweils einen Längsschlitz für unfiltriertes bzw. filtriertes Fluid aufweisen, wobei jeder der Schlitze im Wesentlichen genauso breit ist wie der Filter und im Wesentlichen genauso hoch ist wie die Vorsprünge auf den Einlass- bzw. Auslassseiten des Filters. Der Querschnitt der durch die Vorsprünge gebildeten Durchgangspassagen kann jeweils senkrecht zur Strömungsrichtung des Fluids stehen und kann - betrachtet in Strömungsrichtung - von Reihe zu Reihe abnehmen. Auch können die Vorsprünge, die näher zur Einlassseite des Filters angeordnet sind, größer sein als die Vorsprünge, die näher an der Auslassseite des Filters angeordnet sind. Daneben kann sich auch der Abstand zwischen der Silizium-Platte und der Glas-Platte in dem Bereich von der Einlassseite zur Auslassseite verjüngen. Die Zick-Zack-Konfiguration, die von den wenigstens zwei Reihen von Vorsprüngen gebildet wird, weist einen Neigungswinkel von bevorzugt 20° bis 250°auf. Weitere Einzelheiten dieser Bauteilkonstruktion können der WO-94/07607 entnommen werden.

Schließlich wird die Aufgabe mit einem Zerstäuber zur Abgabe einer bestimmten Menge eines, insbesondere ein Arzneimittel aufweisenden, Fluids als Aerosol mit dem Bauteil der zuvor erläuterten Art gelöst.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispieles unter Bezugnahme auf die zugehörigen Zeichnungen näher erläutert. Es zeigt:

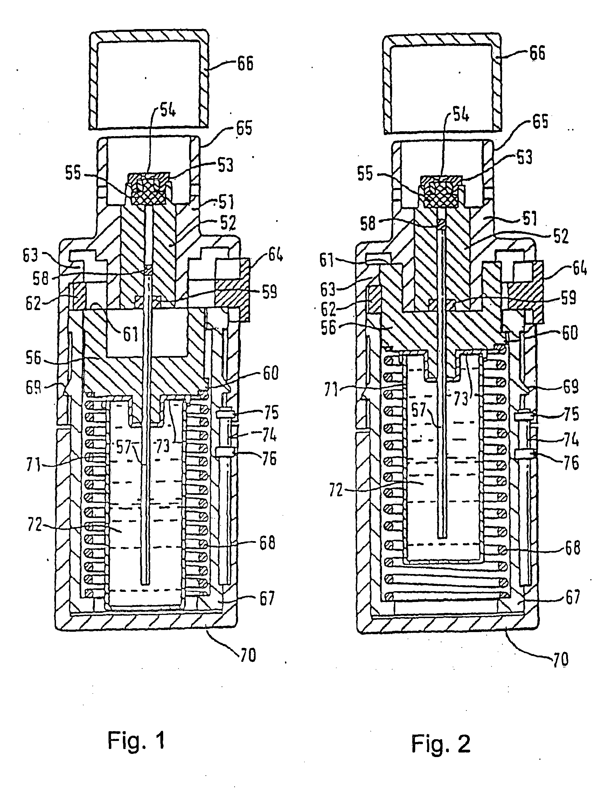

- Fig.1: einen Längsschnitt durch einen erfindungsgemäßen Zerstäuber mit einer gespannten Feder,
- Fig.2: einen Längsschnitt durch den Zerstäuber nach Fig. 1 mit entspannter Feder,

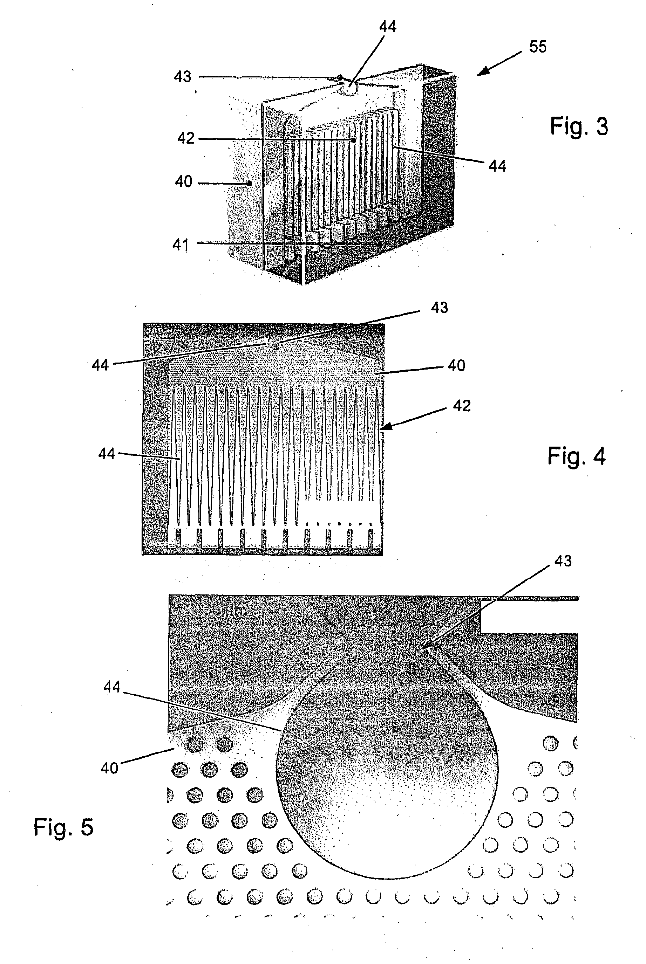

- Fig.3: eine perspektivische Darstellung eines erfindungsgemäßen Bauteils,
- Fig.4: eine Vorderansicht des Bauteils nach Fig. 3 und
- Fig.5: eine vergrößerte Teildarstellung des Bauteils nach Fig. 3.

Ein Gehäuseoberteil 51 des Zerstäubers umfasst ein Pumpengehäuse 52, an dessen Ende ein Halter 53 für eine Zerstäuberdüse angebracht ist. In dem Halter 53 befindet sich eine sich erweiternde Ausnehmung 54 und das als Düsenkörper ausgebildete Bauteil 55. Ein in einem Abtriebsflansch 56 eines Sperrspannwerkes befestigter Hohlkolben 57 ragt teilweise in einen Zylinder des Pumpengehäuses 52 hinein. An seinem Ende trägt der Hohlkolben 57 einen Ventilkörper 58. Der Hohlkolben 57 ist mittels einer Dichtung 59 abgedichtet. Innerhalb des Gehäuseoberteils 51 befindet sich ein Anschlag 60 des Abtriebsflanschs 56, an dem eine Druckfeder 68 anliegt. Nach dem Spannen der Druckfeder 68 schiebt sich ein Sperrglied 62 zwischen einen Anschlag 61 des Abtriebsflanschs 56 und eine Abstützung 63 im Gehäuseoberteil 51. Eine Auslösetaste 64 steht mit dem Sperrglied 62 in Verbindung. Das Gehäuseoberteil 51 endet in einem Mundstück 65 und ist mit einer aufsteckbaren Schutzkappe 66 verschlossen.

Ein Federgehäuse 67 mit der Druckfeder 68 ist mittels Schnappnasen 69 und einem Drehlager an dem Gehäuseoberteil 51 drehbar gelagert. Über das Federgehäuse 67 ist ein Gehäuseunterteil 70 geschoben und innerhalb des Federgehäuses 67 befindet sich ein Vorratsbehälter 71 für zu zerstäubendes Fluid 72. Der Vorratsbehälter 71 ist mit einem Stopfen 73 verschlossen, durch den der Hohlkolben 57 in den Vorratsbehälter 71 hineinragt und mit seinem Ende in das Fluid 72, also den Vorrat an Wirkstofflösung, eintaucht.

In einer Mantelfläche des Federgehäuses 67 ist eine Spindel 74 für ein mechanisches Zählwerk angebracht (optional). An dem dem Gehäuseoberteil 51 zugewandten Ende der Spindel 74 befindet sich ein Antriebsritzel 75. Auf der Spindel 74 sitzt ein Reiter 76.

Das Bauteil 55 - ein so genannter Uniblock - umfasst zwei fest miteinander verbundene Platten 40, 41, von denen die eine Platte 40 aus Silizium fertigt ist und eine Mikrostruktur 42 zur Ausbildung eines Kanal- bzw. Filtersystems und einer sich daran anschließenden Düsenöffnung 43 aufweist. Die Platte 40 aus Silizium ist auf der Seite mit der Mikrostruktur 42 mit der aus Glas gefertigten Platte 41 fest verbunden.

Zumindest die Oberfläche 44 der Mikrostruktur 42 weist eine aus funktionellen Silanen bestehende Beschichtung auf, die eine Wechselwirkung von Bestandteilen und Partikeln der durch die Düsenöffnung 43 ausgetragenen Formulierung mit den Grenzflächen des Bauteils 55 unterbindet. Bei der Verwendung des Zerstäubers mit dem Bauteil 55 werden die Materialrückstände aus dem Bauteil 55 ausgetragen und das beschichtete Bauteil 55 ist durch den Einsatz der monomolekularen, kovalent gebundenen Antihaftschicht selbstreinigend.

Die Oberflächen 44 des Bauteils 55, insbesondere der Mikrostruktur 42, werden zunächst gereinigt, in dem das Bauteil 5 zuerst 5 min in Isopropanol und anschließend 5 min in Wasser getaucht wird. Danach wird das Bauteil 55 für ca. 20 min in einer wässrigen 3% Flusssäurelösung (HF) gebadet.

Die eigentliche Aktivierung der Oberfläche 44 erfolgt mit einem Küvettenreiniger, wobei insbesondere der unter dem Handelsnamen "Hellmanex-Lösung" bekannte Küvettten- oder Glasreiniger der Firma Hellma GmbH & Co. KG, Müllheim, Deutschland, als wässrige 2%ige Lösung bei 70°C für 2x20 min im Ultraschallbecken zum Einsatz kommt.

Zur Funktionalisierung wird beispielsweise 1H,1H,2H,2H-Perfluorooctyltriethoxysilan verwendet, das beispielsweise unter dem Handelsnamen Dynasylan von der Fa. Evonik AG, Düsseldorf, vertrieben wird. Nachdem das Bauteil 55 unter Einwirkung von Ultraschall ca. 2h einer Lösung mit dem funktionellen Silan ausgesetzt ist, lässt man die überschüssige Lösung von dem Bauteil 55 abtropfen und das Bauteil wird ca. 1 bis 2h bei 120°C in einem Ofen getempert. Überschüssige und nicht kovalent gebundene Rückstände werden nach dem Tempern mittels Isopropanol und Wasser von dem Bauteil 55 gewaschen.

## Patentansprüche

1. Verfahren zur Beschichtung einer, insbesondere mikrostrukturierten, Oberfläche (44) eines aus unterschiedlichen Werkstoffen, insbesondere Glas und Silizium, bestehenden Bauteils (55), bei dem zunächst die Oberfläche (44) aktiviert und anschließend beschichtet wird,
**dadurch gekennzeichnet, dass** zur Aktivierung der Oberfläche (44) eine basische Lösung verwendet wird, wobei zur Aktivierung der Oberfläche (44) als basische Lösung ein Küvettenreiniger verwendet wird,
wobei die Oberfläche (44) vor der Aktivierung mittels Isopropanol und Wasser gereinigt wird,
wobei zur Beschichtung der Oberfläche (44) funktionelle Silane in unpolaren, aprotischen oder polaren, protischen Lösungsmitteln verwendet werden, wobei zur Beschichtung der Oberfläche (44) das Bauteil (55) in der Reaktionslösung mit Ultraschall behandelt wird und nach der Beschichtung getrocknet und 1 bis 2h bei 120°C in einem Ofen getempert wird.

2. Verfahren nach Anspruch 1, **dadurch gekennzeichnet, dass** das Bauteil (55) abschließend gewaschen wird.

3. Bauteil, das mindestens zwei fest miteinander verbundene Platten (40, 41) umfasst, von denen wenigstens eine Platte (40) eine Mikrostruktur (42) zur Ausbildung eines Kanal- und/oder Filtersystems und mindestens einer sich daran anschließenden Düsenöffnung (43)aufweist, wobei zumindest die Oberfläche (44) der Mikrostruktur(42) eine Beschichtung aufweist, die in einemVerfahren nach einem der Ansprüche 1 oder 2 aufgebracht ist.

4. Bauteil nach Anspruch 3, **dadurch gekennzeichnet, dass** die eine Platte (40) aus Silizium und die andere Platte (41) aus Glas besteht.

5. Zerstäuber zur Abgabe einer bestimmten Menge eines, insbesondere ein Arzneimittel aufweisenden, Fluids (72) als Aerosol mit einem Bauteil (55) nach Anspruch 3 oder 4.

6. Zerstäuber nach Anspruch 5, **gekennzeichnet durch** ein unteres und ein oberes gegeneinander drehbar gelagertes Gehäuseteil, wobei in dem Gehäuseoberteil (51) ein Federgehäuse (67) mit einer Druckfeder (68) ausgebildet ist, die **durch** Drehen der beiden Gehäuseteile über ein Sperrspannwerk, bevorzugt in Form eines Schraubgewindes oder Getriebes, gespannt und **durch** Drucken eines Auslöseknopfs am Gehäuseoberteil (51) entspannt wird, wobei die Druckfeder (68) einen Abtriebsflansch (56) bewegt, der mit einem Hohlkolben (57) verbunden ist, an dessen unterem Ende ein Behälter aufgesteckt werden kann und an dessen oberem Ende sich ein Ventil und eine Dosierkammer befinden, die mit dem als Düsensystem ausgebildeten Bauteil (55), das im nach oben hin offenen Bereich des Gehäuseoberteils (51) angeordnet ist, in einer eine Flüssigkeit leitenden Verbindung steht.

## Claims

1. Method for coating a surface, particularly a microstructured surface (44) of a component (55) comprising different materials, particularly glass and silicon, wherein the surface (44) is first activated and then coated,
**characterised in that** a basic solution is used to activate the surface (44), a cell cleaner being used as the basic solution for activating the surface,
the surface (44) is cleaned with isopropanol and water before the activation, functional silanes in non-polar, aprotic or polar, protic solvents are used to coat the surface (44), the surface (44) being coated by treating the component (55) with ultrasound in the reaction solution and after the coating the component (55) is dried and heat-treated in an oven for 1 to 2 h at 120°C.

2. Method according to claim 1, **characterised in that** finally the component (55) is washed.

3. Component which comprises at least two plates (40, 41) firmly joined to one another, of which at least one plate (40) has a microstructure (42) for forming a channel and/or filter system and at least one nozzle opening (43) adjacent thereto, while at least the surface (44) of the microstructure (42) has a coating which is applied by a method according to one of claims 1 or 2.

4. Component according to claim 3, **characterised in that** one plate (40) consists of silicon and the other plate (41) consists of glass.

5. Nebuliser for delivering a specific amount of a fluid (72), particularly a fluid comprising a medicament, as an aerosol using a component (55) according to claim 3 or 4.

6. Nebuliser according to claim 5, **characterised by** a lower housing part and an upper housing part mounted to be rotatable against each other, wherein in the upper housing part (51) is formed a spring housing (67) having a compression spring (68) which is tensioned by rotating the two housing parts using a locking clamping mechanism, preferably in the form of a screw thread or gear, and is relaxed by pressing an actuating button on the upper housing part (51), the compression spring (68) moving a power take-off flange (56) which is connected to a hollow piston (57) on the lower end of which a container can be fitted and at the upper end of which are located a valve and a metering chamber which is connected in a fluid-carrying relationship with the component (55) embodied as a nozzle system, said component (55) being arranged in the upwardly open region of the upper housing part (51).

## Revendications

1. Procédé de revêtement d'une surface (44), en particulier microstructurée, d'un composant (55) constitué de diverses substances, en particulier de verre et de silicium, dans le cadre duquel la surface (44) est activée dans un premier temps avant d'être revêtue,
**caractérisé en ce qu'**on utilise aux fins de l'activation de la surface (44) une solution basique, un détergent de cuvette étant utilisé comme solution basique aux fins de l'activation de la surface (44), la surface (44) étant nettoyée à l'isopropanol et à l'eau avant l'activation,
des silanes fonctionnels étant utilisés dans des solvants non polaires, aprotiques ou polaires, protiques aux fins du revêtement de la surface (44), le composant (55) étant traité aux ultrasons dans la solution réactionnelle aux fins du revêtement de la surface (44), étant séché après l'opération de revêtement et trempé dans un four à une température de 120°C pendant 1 à 2 heures.

2. Procédé selon la revendication 1, **caractérisé en ce que** le composant (55) est pour finir lavé.

3. Composant, comprenant au moins deux plaques (40, 41) reliées l'une à l'autre de manière fixe, parmi lesquelles au moins une plaque (40) présente une microstructure (42) servant à réaliser un système de canal et/ou de filtre et à réaliser au moins une ouverture de buse (43) adjacente audit système, au moins la surface (44) de la microstructure (42) présentant un revêtement, qui est appliqué lors d'un procédé selon l'une quelconque des revendications 1 ou 2.

4. Composant selon la revendication 3, **caractérisé en ce que** l'une (40) des plaques est constituée de silicium et l'autre plaque (41) est constituée de verre.

5. Pulvérisateur servant à distribuer une certaine quantité d'un fluide (72), présentant en particulier un médicament, sous la forme d'un aérosol, comprenant un composant (55) selon la revendication 3 ou 4.

6. Pulvérisateur selon la revendication 5, **caractérisé par** une partie inférieure de boîtier et une partie supérieure de boîtier logée de manière à pouvoir tourner par rapport à la partie inférieure de boîtier, un boîtier à ressort (67) comprenant un ressort de compression (68) étant réalisé dans la partie supérieure de boîtier (51), lequel ressort de compression est tendu du fait de la rotation des deux parties de boîtier par l'intermédiaire d'un mécanisme tendeur à blocage, se présentant de préférence sous la forme d'un filet de vissage ou d'un mécanisme, et est détendu du fait d'une pression sur un bouton de déclenchement au niveau de la partie supérieure de boîtier (51), le ressort de compression (68) déplaçant une flasque de sortie (56), qui est reliée à un piston creux (57), au niveau de l'extrémité inférieure duquel peut être enfiché un récipient et au niveau de l'extrémité supérieure duquel se trouvent une soupape et une chambre de dosage, qui est en liaison de conduction de liquide avec le composant (55) réalisé sous la forme d'un système de buse et disposé dans la zone, ouverte en direction du haut, de la partie supérieure de boîtier (51).
